(19) Europäisches Patentamt
European Patent Office
Office européen des brevets

(11) **EP 2 823 750 A1**

(12) **EUROPEAN PATENT APPLICATION**
published in accordance with Art. 153(4) EPC

(43) Date of publication:
**14.01.2015 Bulletin 2015/03**

(51) Int Cl.:
***A61B 1/06*** *(2006.01)*

(21) Application number: **13757710.2**

(86) International application number:
**PCT/JP2013/056187**

(22) Date of filing: **06.03.2013**

(87) International publication number:
**WO 2013/133341 (12.09.2013 Gazette 2013/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.03.2012 US 201261607834 P**

(71) Applicant: **Olympus Corporation**
**Shibuya-ku**
**Tokyo 151-0072 (JP)**

(72) Inventors:
• **SHONO, Yuki**
 **Tokyo 151-0072 (JP)**
• **ITO, Ryosuke**
 **Tokyo 151-0072 (JP)**
• **TAKAOKA, Hideyuki**
 **Tokyo 151-0072 (JP)**

(74) Representative: **Gunzelmann, Rainer**
**Wuesthoff & Wuesthoff**
**Patent- und Rechtsanwälte**
**Schweigerstraße 2**
**81541 München (DE)**

(54) **OPTICAL MEASURING DEVICE**

(57)     An object of the present invention is to provide an optical measurement device that can measure a living tissue at an S/N ratio maintained without increasing the length of the measurement time. An optical measurement device 3 includes: an illumination control unit 332a that causes a measurement light source unit 321 to execute alternately emission and stoppage of measurement light within the time from the start to end of the measurement by the optical measurement device 3, and causes the measurement light source unit 321 to emit the measurement light such that the emission time during which the measurement light source unit 321 emits the measurement light is longer than the stoppage time during which the measurement light source unit 321 stops the meas-urement light; and a computation unit 332b that removes, from an intensity of return light of the measurement light detected by a first detection unit 325, a detected intensity of return light of observation light from the endoscope device and reflected from a living tissue S1.

FIG.2

**Description**

Field

The present invention relates to an optical measurement device that radiates measurement light to a living tissue and estimates characterization of the living tissue based on a measured value of return light of the measurement light reflected and/or scattered from the living tissue.

Background

There has been conventionally known an optical measurement device that radiates measurement light to a living tissue and estimates characterization of the living tissue based on a measured value of detection light reflected or scattered from the living tissue. The optical measurement device is used in combination with an endoscope for observing body organs such as a digestive system. As such an optical measurement device, there has been suggested an optical measurement device using LEBS (low-coherence enhanced backscattering) to detect characterization of a living tissue by radiating low-coherent white light with a short spatial coherent length to the living tissue from a distal end of an radiation fiber of a probe and measuring intensity distribution of scattered light at a plurality of angles by the use of a plurality of light-receiving fibers.

For such an optical measurement device, there has been known a technique for optical measurement of in-vivo mucous membranes using an endoscope (refer to Patent Literature 1). This technique makes it possible to stop radiation of observation light from the endoscope while optical measurement is made with a measurement probe, thereby to prevent that the observation light from the endoscope is measured by the measurement probe.

There has also been known a technique for computing a measured value of a target to be measured by subtracting a measured value detected by a detection unit when an optical measurement device radiates no measurement light from a measured value detected by the detection unit when the optical measurement device radiates measurement light under illumination of observation light from an endoscope (refer to Patent Literature 2).

Citation List

Patent Literatures

Patent Literature 1: Japanese Patent Application Laid-open No. 9-248281
Patent Literature 2: Japanese Patent Application Laid-open No. 2000-14629

Summary

Technical Problem

According to the foregoing Patent Literature 1, however, when radiation of the observation light from the endoscope is stopped, the observation image of the target to be measured becomes dark during the stoppage. This makes it difficult to align the position of the measurement probe with a desired position in the target to be measured.

According to the foregoing Patent Literature 2, time is allocated between normal observation and optical measurement. Thus, measurement time from start to end of measurement needs to be twice as longer as time for actually performing optical measurement. If the measurement time is shortened, there is a problem that the amount of measurement information becomes smaller to reduce the S/N ratio.

The present invention is devised in light of the foregoing circumstances. An object of the present invention is to provide an optical measurement device that makes it possible to prevent that the observation image of a target to be measured becomes dark and perform optical measurement of the target to be measured at an S/N ratio maintained without increasing the length of the measurement time.

Solution to Problems

To solve the above-described problem and achieve the object, an optical measurement device according to the present invention is configured to be inserted into an inside of a subject via an insertion portion of an endoscope radiating observation light for observing a target from a distal end of the insertion portion to be inserted into the inside of the subject to shoot the inside of the subject, and to measure optical characteristics of a living tissue in the subject.

The optical measurement device includes: a measurement light source unit that emits measurement light including at least part of a wavelength band of the observation light radiated by the endoscope; a measurement probe including an illumination fiber configured to radiate the measurement light emitted from the measurement light source unit to the living tissue, and a light-receiving fiber configured to receive return light of the measurement light reflected and/or scattered from the living tissue; a detection unit that detects the return light of the measurement light received by the light-receiving fiber; an illumination control unit that causes the measurement light source unit to execute alternately emission and stoppage of the measurement light within the time from the start to end of the measurement by the optical measurement device, and causes the measurement light source unit to emit the measurement light such that the emission time during which the measurement light source unit emits the measurement light is longer than the stoppage time during which the measurement light source unit stops the measurement light; and a computation unit that removes, from an intensity of return light of the measurement light detected by the detection unit, a detected intensity of return light of the observation light radiated by the endoscope device and reflected from the living tissue, wherein the computation unit computes a detected intensity I_C in which the detected intensity of return light of the observation light is removed from the detected intensity of return light of the measurement light by the following equation (1):

$$\mathrm{I\_C \ = \ I\_t_{on} \ - \ I\_t_{off} \ \times \ (t_{on}/t_{off}) \qquad \ldots \ (1)}$$

where $t_{on}$ denotes the emission time, $t_{off}$ denotes the stoppage time, $I\_t_{on}$ denotes the intensity detected by the detection unit during the emission time, and $I\_t_{off}$ denotes the intensity detected by the detection unit during the stoppage time.

Moreover, in the above-described optical measurement device according to the present invention, the illumination control unit performs variable control on the emission time and the stoppage time according to the type of the living tissue.

Moreover, in the above-described optical measurement device according to the present invention, the illumination control unit causes the measurement light source unit to execute alternately emission and stoppage of the measurement light, and the computation unit computes the average value of a plurality of intensities repeatedly detected by the detection unit.

Moreover, in the above-described optical measurement device according to the present invention, the optical characteristics are a spectrum of return light of the measurement light, and the detection unit has a spectroscope that detects the spectrum of return light of the measurement light.

Moreover, in the above-described optical measurement device according to the present invention, the illumination control unit causes the measurement light source unit to emit the measurement light during the emission time and the stoppage time where the intensity detected by the detection unit during the emission time and the detected intensity of return light of the observation light from the endoscope are equal in S/N ratio.

Moreover, in the above-described optical measurement device according to the present invention, the light-receiving fiber includes a plurality of light-receiving channels that receive return light of the measurement light at different angles, and the detection unit includes a plurality of detection units that detect the return light of the measurement light received by the plurality of light-receiving channels.

Advantageous Effects of Invention

According to the optical measurement device of the present invention, it is possible to produce the advantages of preventing that the observation image of a target to be measured becomes dark and allowing optical measurement of the target to be measured at an S/N ratio maintained without increasing the length of the measurement time.

Brief Description of Drawings

FIG. 1 is a diagram illustrating a schematic configuration of an endoscope system according to a first embodiment of the present invention.
FIG. 2 is a block diagram illustrating a schematic configuration of an optical measurement device, an endoscope light source device, and a control device in the endoscope system according to the first embodiment of the present invention.
FIG. 3 is a diagram illustrating a radiation timing for the optical measurement device to radiate measurement light according to the first embodiment of the present invention.
FIG. 4 is a diagram illustrating an intensity detected by a first detection unit of the optical measurement device during an emission time of a measurement light source unit according to the first embodiment of the present invention.

FIG. 5 is a diagram illustrating an intensity detected by the first detection unit of the optical measurement device during a stoppage time of the measurement light source unit according to the first embodiment of the present invention.

FIG. 6 is a diagram illustrating results of computation by a computation unit of the optical measurement device according to the first embodiment of the present invention.

FIG. 7 is a block diagram illustrating a schematic configuration of an optical measurement device, an endoscope light source device, and a control device in an endoscope system according to a modification example 1 of the first embodiment of the present invention.

FIG. 8 is a block diagram illustrating a schematic configuration of an optical measurement device, an endoscope light source device, and a control device in an endoscope system according to a second embodiment of the present invention.

FIG. 9 is a diagram illustrating a schematic configuration of a rotary filter of the optical measurement device according to the second embodiment of the present invention.

FIG. 10 is a diagram illustrating radiation timings for the optical measurement device to radiate measurement light during observation of a subject according to the second embodiment of the present invention.

FIG. 11 is a block diagram illustrating a schematic configuration of an optical measurement device, an endoscope light source device, and a control device in an endoscope system according to a third embodiment of the present invention.

FIG. 12 is a diagram illustrating a schematic spectrum detected during an emission time of a measurement light source unit by a first spectroscope of the optical measurement device according to the third embodiment of the present invention.

FIG. 13 is a diagram illustrating a schematic spectrum detected during a stoppage time of the measurement light source unit by the first spectroscope of the optical measurement device according to the third embodiment of the present invention.

FIG. 14 is a diagram illustrating a schematic spectrum of return light of illumination light radiated from the endoscope light source device computed by a computation unit of the optical measurement device according to the third embodiment of the present invention.

FIG. 15 is a diagram illustrating a schematic spectrum of only measurement light computed by the computation unit of the optical measurement device according to the third embodiment of the present invention.

FIG. 16 is a block diagram illustrating a schematic configuration of an optical measurement device, an endoscope light source device, and a control device in an endoscope system according to a fourth embodiment of the present invention.

FIG. 17 is a diagram illustrating emission timings for the optical measurement device to radiate measurement light according to the fourth embodiment of the present invention. Description of Embodiments

Examples of an optical measurement device using LEBS technique will be described below in detail with reference to the drawings, as preferred embodiments of an optical measurement device and an endoscope system according to the present invention. The present invention is not limited to the embodiments. In the description of the drawings, the same components are given the same reference numerals. It is to be noted that the drawings are schematic and the relationship between thickness and width of respective members and the ratios among the members illustrated in the drawings may differ from actual ones. In addition, the dimensions and ratios may be differently illustrated among the drawings.

(First embodiment)

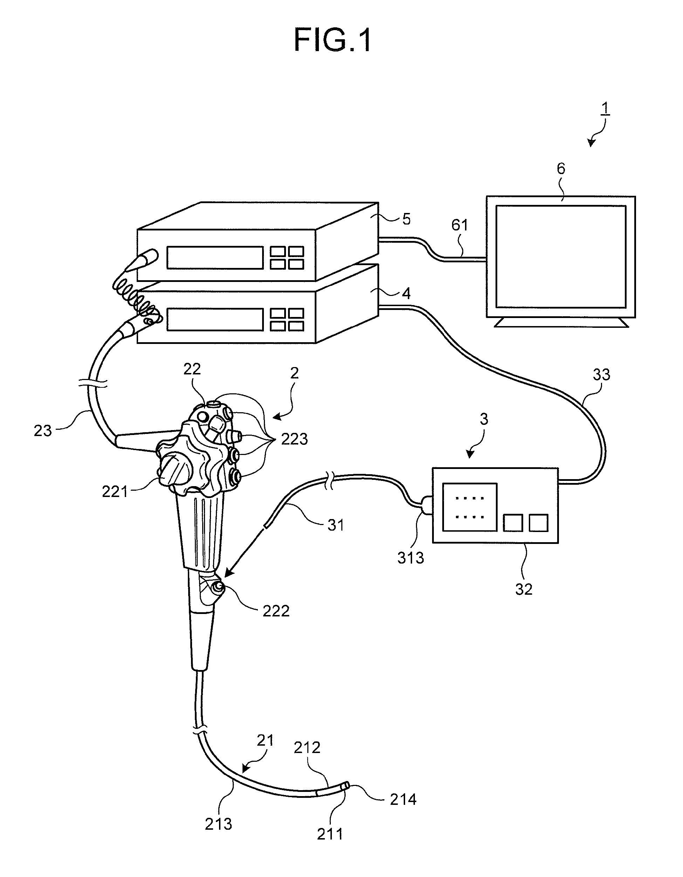

FIG. 1 is a diagram illustrating a schematic configuration of an endoscope system according to a first embodiment of the present invention. An endoscope system 1 illustrated in FIG. 1 includes: an endoscope device 2 (endoscope) that is inserted into a subject to shoot the inside of the subject and generate an image signal of the inside of the subject; an optical measurement device 3 that is introduced into the subject via the endoscope device 2 to estimate characterization of a living tissue in the subject; an endoscope light source device 4 that generates illumination light of the endoscope device 2; a control device 5 (processor) that performs predetermined image processing on the image signal captured by the endoscope device 2 and controls components of the endoscope system 1; and a display device 6 that displays an image corresponding to the image signal on which the control device 5 has performed the image processing.

The endoscope device 2 includes an insertion unit 21 that is inserted into the subject, an operating unit 22 that is located at a proximal end side of the insertion unit 21 and is held by an operator, and a flexible universal cord 23 that is extended from a side portion of the operating unit 22.

The insertion unit 21 is composed of an illumination fiber (light guide cable) and an electric cable or the like. The insertion unit 21 has a distal end portion 211 having an imaging unit with a CCD sensor or a CMOS sensor or the like embedded therein as an imaging element for capturing the inside of the subject; a curve portion 212 that is capable of free curve and formed by a plurality of curve pieces; and a flexible tube portion 213 that has flexibility and is provided at a proximal end portion side of the curve portion 212. The distal end portion 211 is provided with an illumination unit that radiates the inside of the subject via an illumination lens, an observation unit that captures the inside of the subject, an opening portion 214 that communicates with a treatment tool channel, and an air/water feeding nozzle (not illustrated).

The operating unit 22 has a curve knob 221 that curves the curve portion 212 in vertical and horizontal directions; a treatment tool insertion portion 222 into which a treatment tool such as a biopcy forceps, a laser knife, a measurement probe of the optical measurement device 3, or the like is inserted; and a plurality of switch portions 223 that are used to operate peripheral devices such as the optical measurement device 3, the endoscope light source device 4, the control device 5, an air feeding device, a water feeding device, a gas feeding device and the like. The treatment tool inserted from the treatment tool insertion portion 222 lets itself out from the opening portion 214 at the distal end of the insertion unit 21 through the treatment tool channel provided inside.

The flexible universal cord 23 is composed of an illumination fiber and an electric cable or the like. The flexible universal cord 23 transfers illumination light emitted from the endoscope light source device 4 to the distal end portion 211 via the operating unit 22 and the flexible tube portion 213. The flexible universal cord 23 transfers an image signal captured by the imaging unit such as an imaging element provided at the distal end portion 211 to the control device 5.

The optical measurement device 3 includes: a measurement probe 31 that is inserted into the inside of the subject through the treatment tool insertion portion 222 of the endoscope device 2; a main body unit 32 that outputs measurement light to the measurement probe 31 and receives return light of the measurement light reflected and/or scattered from a target to be measured via the measurement probe 31 to estimate characterization (characteristic values) of the target to be measured; and a transfer cable 33 that transfers results of measurement and the like by the main body unit 32 to the control device 5.

The endoscope light source device 4 supplies illumination light (observation light) to the endoscope device 2 connected via the illumination fiber of the flexible universal cord 23. The endoscope light source device 4 is composed of a white light source or a special light source or the like.

The control device 5 performs predetermined image processing on the image signal of the inside of the subject captured by the distal end portion 211 of the endoscope device 2 and transferred via the flexible universal cord 23, and outputs the image signal to the display device 6. The control device 5 controls the components of the endoscope system 1 based on various instructive signals transmitted from the switch portions 223 of the operating unit 22 in the endoscope device 2 via the flexible universal cord 23.

The display device 6 displays the image corresponding to the image signal on which the control device 5 has performed the predetermined image processing and results of measurement by the optical measurement device 3 and the like, through an image cable 61. The display device 6 is composed of a liquid crystal or organic EL (electro luminescence) display or the like. This allows the operator to operate the endoscope device 2 while viewing the image displayed on the display device 6 to observe a desired position in the subject and determine characterization of the subject at the position.

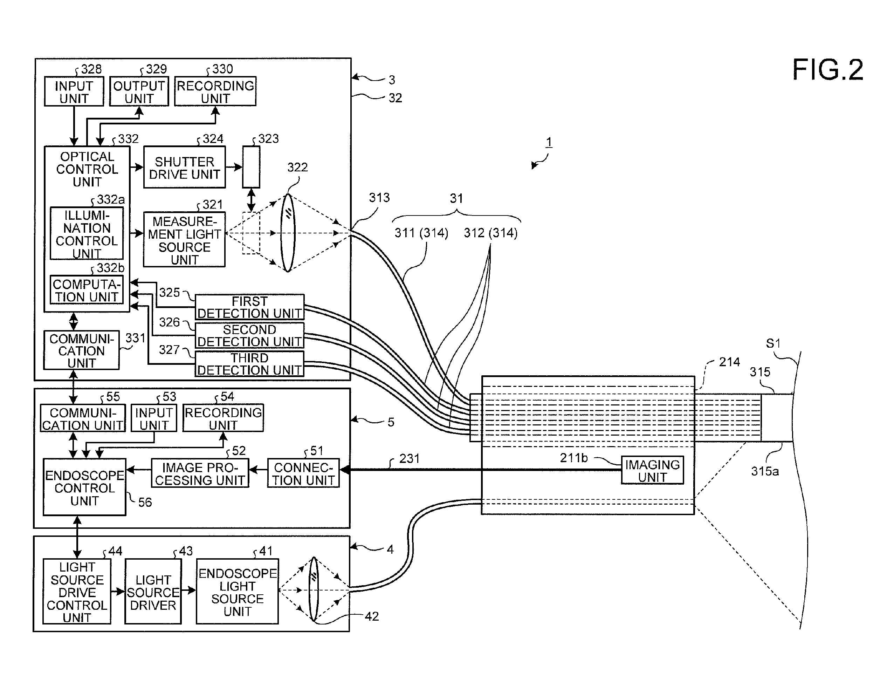

Next, detailed configurations of the optical measurement device 3, the endoscope light source device 4, and the control device 5 explained above with reference to FIG. 1 will be described. FIG. 2 is a block diagram illustrating a schematic configuration of the optical measurement device 3, the endoscope light source device 4, and the control device 5 illustrated in FIG. 1.

First, a detailed configuration of the optical measurement device 3 will be descried. The optical measurement device 3 includes the measurement probe 31 and the main body unit 32.

The measurement probe 31 is composed of one or more optical fibers or the like. Specifically, the measurement probe 31 is composed of an illumination fiber 311 that emits measurement light (illumination light) to a living tissue S1 as a target to be measured, and a plurality of light-receiving fibers 312 (for example, a first light-receiving channel, a second light-receiving channel, and a third light-receiving channel) into which return light of the measurement light reflected and/or scattered from the target to be measured is entered at different angles (scatter angles). The illumination fiber 311 and the light-receiving fibers 312 are arranged in parallel to each other at least at their distal ends. The measurement probe 31 has a proximal end portion 313, a flexible portion 314, and a distal end portion 315.

The proximal end portion 313 is detachably connected to a connector portion (not illustrated) of the main body unit 32. The flexible portion 314 has flexibility, and transfers measurement light emitted from the main body unit 32 to the distal end portion 315 including a proximal end of the illumination fiber 311 with an end surface exposed, and transfers to the main body unit 32 return light of the measurement light entered via the distal end portion 315. The distal end portion 315 emits the measurement light transferred from the flexible portion 314 to the living tissue S1, and receives entry of the return light of the measurement light reflected and/or scattered from the living tissue S1. The distal end portion 315 is provided with a permeable rod 315a as an optical member. The rod 315a is cylindrical in shape such that a distance from the surface of the living tissue S1 to the distal ends of the illumination fiber 311 and the light-receiving fibers 312 becomes uniform. Although, in FIG. 2, the measurement probe 31 has the three light-receiving fibers 312 as an example, the number of the light-receiving fibers 312 may be two or more because the measurement probe 31 only needs to receive at least two or more scattered lights at different scatter angles. In addition, the number of the illumination fiber 311 can also be changed as appropriate according to the kind and region of the living tissue S1.

The main body unit 32 includes a measurement light source unit 321, a condenser lens 322, a shutter 323, a shutter drive unit 324, a first detection unit 325, a second detection unit 326, a third detection unit 327, an input unit 328, an output unit 329, a recording unit 330, a communication unit 331, and an optical control unit 332.

The measurement light source unit 321 emits incoherent measurement light including at least part of a wavelength band of observation light radiated by the endoscope device 2 to the illumination fiber 311. The measurement light source unit 321 is composed of an incoherent light source such as a white LED (light emitting diode), a xenon lamp, a tungsten lamp, and a halogen lamp, and a light source driver and the like. The measurement light source unit 321 emits the measurement light to the illumination fiber 311 under control of the optical control unit 332.

The condenser lens 322 is composed of one or more lenses and concentrates the observation light emitted from the measurement light source unit 321 to the proximal end portion 313 of the illumination fiber 311.

The first detection unit 325 detects return light of the measurement light radiated from the illumination fiber 311 of the measurement probe 31 and reflected and/or scattered from the living tissue S1, and outputs the results of detection to the optical control unit 332. Specifically, the first detection unit 325 detects the intensity of the return light of the measurement light entered from the light-receiving fibers 312 of the measurement probe 31, and outputs the results of detection to the optical control unit 332. The first detection unit 325 is composed of a light-receiving sensor such as a CCD or a CMOS.

The second detection unit 326 and the third detection unit 327 are implemented by the same configuration as that of the first detection unit 325, and thus detects the return light of the measurement light radiated from the measurement probe 31, entered to the living tissue S1 via the measurement probe 31, and reflected and/or scattered from the living tissue S1, and then outputs the results of detection to the optical control unit 332.

The input unit 328 receives input of an instructive signal for instructing activation of the optical measurement device 3 and an instruction for starting of optical measurement by the optical measurement device 3 and the like, and outputs the instructions to the optical control unit 332. The input unit 328 is composed of push switches, touch panels, and the like.

The output unit 329 outputs the results of measurement by the optical measurement device 3 and information on various processes performed by the optical measurement device 3. The output unit 329 is composed of a liquid crystal display or organic EL display, and a speaker or the like.

The recording unit 330 records various programs for operating the optical measurement device 3, various data and parameters for use in optical measurement processes. The recording unit 330 temporarily records information in processing by the optical measurement device 3 and the results of measurement of the living tissue S1. The recording unit 330 is composed of a volatile memory and a non-volatile memory or the like. The recording unit 330 may be a memory card detachably attached to the main body unit 32.

The communication unit 331 is a communication interface for communication with the control device 5 via the transfer cable 33 (refer to FIG. 1). The communication unit 331 outputs the results of measurement by the optical measurement device 3 to the control device 5 and outputs instructive signals and control signals transmitted from the control device 5 to the optical control unit 332.

The optical control unit 332 is composed of a CPU (central processing unit) and the like, and controls processing operations by the components of the optical measurement device 3. The optical control unit 332 performs centralized control of operations of the optical measurement device 3 by transferring instructive information and data to the components of the optical measurement device 3. The optical control unit 332 has an illumination control unit 332a and a computation unit 332b.

The illumination control unit 332a temporally controls the intensity of the measurement light radiated from the measurement light source unit 321 to the living tissue S1. Specifically, the illumination control unit 332a causes the measurement light source unit 321 to execute alternately emission and stoppage of the measurement light within the time from the start to end of the measurement by the optical measurement device 3, and causes the measurement light source unit 321 to emit the measurement light such that the emission time during which the measurement light source unit 321 emits the measurement light is longer than the stoppage time during which the measurement light source unit 321 stops the measurement light.

The computation unit 332b computes optical characteristics relative to characterization of the living tissue S1, based on the results of computation input from the first detection unit 325, the second detection unit 326, and the third detection unit 327. The computation unit 332b also remove, from the intensities of return light of the measurement light detected by the first detection unit 325, the second detection unit 326, and the third detection unit 327, the detected intensity of return light of the observation light radiated to the living tissue S1 by the endoscope device 2 and reflected from the living tissue S1, thereby to compute the detected intensity of only the measurement light emitted from the measurement light source unit 321.

Next, the endoscope light source device 4 will be described. The endoscope light source device 4 includes an endoscope light source unit 41, a condenser lens 42, a light source driver 43, and a light source drive control unit 44.

The endoscope light source unit 41 generates observation light (illumination light) to be supplied to the endoscope device 2. The endoscope light source unit 41 is composed of a white LED or a xenon lamp or the like.

The condenser lens 42 is arranged on a light path of white light generated by the endoscope light source unit 41 to collect the observation light generated by the endoscope light source unit 41 and emit the same to the illumination fiber 311 as a light guide cable of the flexible universal cord 23.

The light source driver 43 supplies predetermined electric power to the endoscope light source unit 41 under control of the light source drive control unit 44.

The light source drive control unit 44 controls driving of the endoscope light source unit 41 under control of the control device 5. Specifically, the light source drive control unit 44 controls the amount of electric current to be supplied from the light source driver 43 to the endoscope light source unit 41 and radiation timing, based on drive signals input from the control device 5. The light source drive control unit 44 is composed of a CPU or the like.

Next, the control device 5 will be described. The control device 5 includes a connection unit 51, an image processing unit 52, an input unit 53, a recording unit 54, a communication unit 55, and an endoscope control unit 56.

The connection unit 51 is connected to an electric cable (communication cable) 231 of the flexible universal cord 23. The connection unit 51 receives an image signal as a digital signal captured by an imaging unit 211b arranged in the vicinity of an observation window (not illustrated) of the distal end portion 211 via the electric cable 231 of the flexible universal cord 23, and outputs the same to the image processing unit 52.

The image processing unit 52 performs predetermined image processing on the image signal output from the connection unit 51, and outputs the same to the display device 6. Specifically, the image processing unit 52 performs on the image signal (image data) image processing including at least optical black subtraction, white balance (WB) adjustment, color matrix computation, gamma correction, color reproduction, and edge enhancement, and the like. The image processing unit 52 converts the digital image signal having undergone the image processing into an analog signal, and outputs the converted analog image signal in a high-vision format or the like to the display device 6. Accordingly, the display device 6 displays one in-vivo image.

The input unit 53 receives input of various instructive signals from the endoscope system 1. Specifically, the input unit 53 receives input of various kinds of information such as subject information, identification information of the endoscope device 2, and inspection contents. The input unit 53 is composed of operation devices such as a mouse, a keyboard, and a touch panel.

The recording unit 54 records various programs for operating the control device 5 and the endoscope light source device 4. The recording unit 54 temporarily records information in processing by the control device 5. The recording unit 54 records the image signal on which the image processing unit 52 has performed image processing and the results of measurement by the optical measurement device 3. The recording unit 54 is composed of a volatile memory and a non-volatile memory. The recording unit 54 may be composed of a memory card or the like externally attached to the control device 5.

The communication unit 55 is a communication interface for communication with the optical measurement device 3 via the transfer cable 33.

The endoscope control unit 56 controls processing operations by the components of the control device 5. The endoscope control unit 56 controls operations of the control device 5 by transferring instructive information and data to the components of the control device 5. The endoscope control unit 56 is connected to the endoscope device 2, the optical measurement device 3, and the endoscope light source device 4 via cables. The endoscope control unit 56 is implemented by using a CPU or the like.

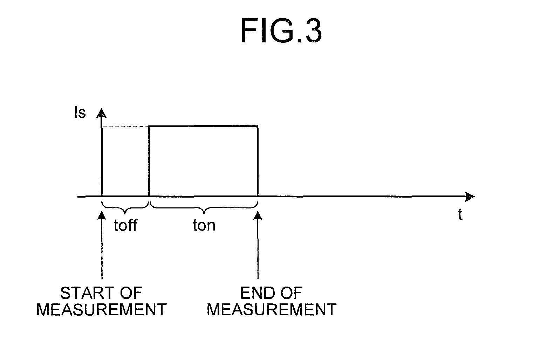

In the thus configured endoscope system 1, a radiation timing for the optical measurement device 3 to radiate the measurement light during observation of the subject and a method for computation by the computation unit 332b will be described. FIG. 3 is a diagram illustrating a radiation timing for the optical measurement device 3 to radiate the measurement light. Referring to FIG. 3, the horizontal axis indicates time (t), and the vertical axis indicates intensity $I_s$ of measurement light from the measurement light source unit 321. FIG. 3 illustrates the intensity of the measurement light with a predetermined wavelength.

As illustrated in FIG. 3, the illumination control unit 332a causes the measurement light source unit 321 to execute alternately emission and stoppage of the measurement light within a period of measurement time for measurement of the living tissue S1. In addition, the illumination control unit 332a controls the emission timing of the measurement light source unit 321 such that emission time $t_{on}$ during which the measurement light source unit 321 emits the measurement light is longer than stoppage time $t_{off}$ during which the measurement light source unit 321 stops the measurement light.

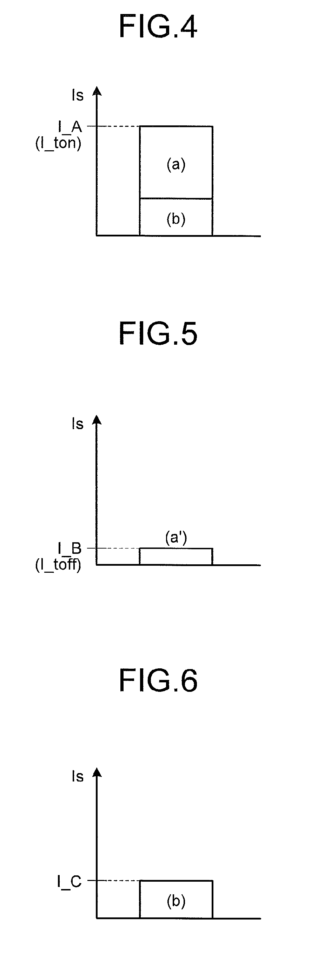

Next, a method for computation by the computation unit 332b will be described. FIG. 4 is a diagram illustrating an intensity detected by the first detection unit 325 during the emission time of the measurement light source unit 321. FIG. 5 is a diagram illustrating an intensity detected by the first detection unit 325 during the stoppage time of the measurement light source unit 321. FIG. 6 is a diagram illustrating results of computation by the computation unit 332b. In FIGS. 4 to 6, the vertical axis indicates detected intensity $I_s$.

As illustrated in FIGS. 4 and 5, first, the computation unit 332b uses intensity $I\_t_{on}$ detected by the first detection unit 325 during the emission time $t_{on}$ of the measurement light source unit 321 and intensity $I\_t_{off}$ detected by the first detection unit 325 during the stoppage time $t_{off}$ of the measurement light source unit 321 to compute, of the light detected by the first detection unit 325 during the emission time $t_{on}$ of the measurement light source unit 321, detected intensity $I\_B$ of a return light component of the observation light from the endoscope light source unit 41 by the following equation (1):

$$I\_B = I\_t_{off} \times (t_{on}/t_{off}) \hspace{3cm} (1)$$

Subsequently, as illustrated in FIG. 6, the computation unit 332b computes detected intensity $I\_C$ of return light of only the measurement light from the measurement light source unit 321 by the following equation (2) :

$$I\_C = I\_t_{on} - I\_t_{off} \times (t_{on}/t_{off}) \hspace{2.5cm} (2)$$
$$= I\_t_{on} - I\_B$$

As described above, the computation unit 332b uses the foregoing equations (1) and (2) to compute the detected intensity of return light of only the measurement light from the measurement light source unit 321. The computation unit 332b performs the same computation with respect to the second detection unit 326 and the third detection unit 327.

According to first embodiment of the present invention descried above, even if the illumination control unit 332a sets the emission time $t_{on}$ to be longer than the stoppage time $t_{off}$ of the measurement light source unit 321, it is possible to precisely perform quantitative optical characteristics of the living tissue S1.

In addition, according to the first embodiment of the present invention, actual time for measurement of optical characteristics can be made relatively longer in the entire measurement time, thereby increasing the S/N ratio during the measurement.

In the first embodiment of the present invention, the illumination control unit 332a may control variably the emission time during which the measurement light source unit 321 emits the measurement light and the stoppage time during which the measurement light source unit 321 stops the measurement light, depending on the type of the living tissue S1 or the position of the target to be observed. For example, the illumination control unit 332a may set the emission time to be twice as longer as the stoppage time. Accordingly, it is possible to measure precise optical characteristics for each of the regions in the target to be measured.

(Modification example 1 of the first embodiment)

Next, modification example 1 of the first embodiment of the present invention will be described. The modification example 1 of the first embodiment is different from the first embodiment in the configuration of the optical measurement device of the endoscope system 1. Thus, hereinafter, the configuration of the optical measurement device in the modification example 1 of the first embodiment will be described. In the modification example 1 of the first embodiment, the same components as those in the endoscope system 1 according to the first embodiment will be given the same reference numerals as those in the first embodiment, and descriptions thereof will be omitted.

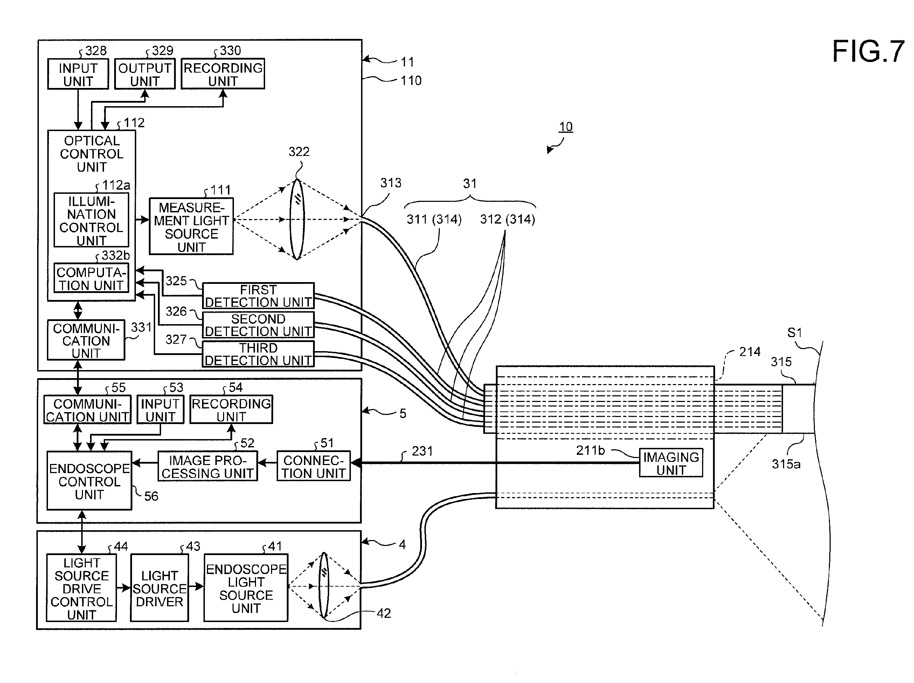

FIG. 7 is a block diagram illustrating a schematic configuration of an optical measurement device, an endoscope light source device, and a control device in an endoscope system according to the modification example 1 of the first embodiment of the present invention. An endoscope system 10 illustrated in FIG. 7 includes the endoscope light source device 4, the control device 5, and an optical measurement device 11.

The optical measurement device 11 includes the measurement probe 31 and a main body unit 110. The main body unit 110 includes a measurement light source unit 111, an optical control unit 112, the condenser lens 322, the first detection unit 325, the second detection unit 326, the third detection unit 327, the input unit 328, the output unit 329, the recording unit 330, and the communication unit 331.

The measurement light source unit 111 emits incoherent measurement light to be radiated to the living tissue S1, to the illumination fiber 311. The measurement light source unit 111 is composed of a white LED and an LED driver or the like. The measurement light source unit 111 emits the measurement light under control of the optical control unit 112.

The optical control unit 112 is composed of a CPU or the like, and controls processing operations by the components of the optical measurement device 11. The optical control unit 112 performs centralized control on operations of the optical measurement device 11 by transferring instructive information and data to the components of the optical measurement device 11. The optical control unit 112 has an illumination control unit 112a and the computation unit 332b.

The illumination control unit 112a temporally controls the intensity of the measurement light radiated from the measurement light source unit 111 to the living tissue S1. Specifically, the illumination control unit 112a causes the measurement light source unit 111 to execute alternately emission and stoppage of the measurement light within the time from the start to end of the measurement by the optical measurement device 11, and causes the measurement light source unit 111 to emit the measurement light such that the emission time during which the measurement light source unit 111 emits the measurement light is longer than the stoppage time during which the measurement light source unit 111 stops the measurement light.

The thus configured optical measurement device 11 causes the measurement light source unit 111 to emit the measurement light toward the illumination fiber 311 such that the emission time of the illumination control unit 112a is longer than the stoppage time of the illumination control unit 112a.

According to the modification example 1 of the first embodiment of the present invention described above, even if the stoppage time during which the measurement light source unit 111 stops the measurement light is shortened, it is possible to precisely perform quantitative optical characteristics.

In addition, according to the modification example 1 of the first embodiment of the present invention, actual time for measurement of optical characteristics can be made relatively longer in the entire measurement time, thereby increasing the S/N ratio during the measurement.

(Second embodiment)

Next, a second embodiment of the present invention will be described. The second embodiment is different from the first embodiment described above in configuration and illumination method of the optical measurement device. Thus, in hereinafter, the configuration of the optical measurement device according to the second embodiment will be described, and then the illumination method executed by the optical measurement device according to the second embodiment will be described. The same components as those in the first embodiment described above will be given the same reference numerals as those in the first embodiment, and descriptions thereof will be omitted.

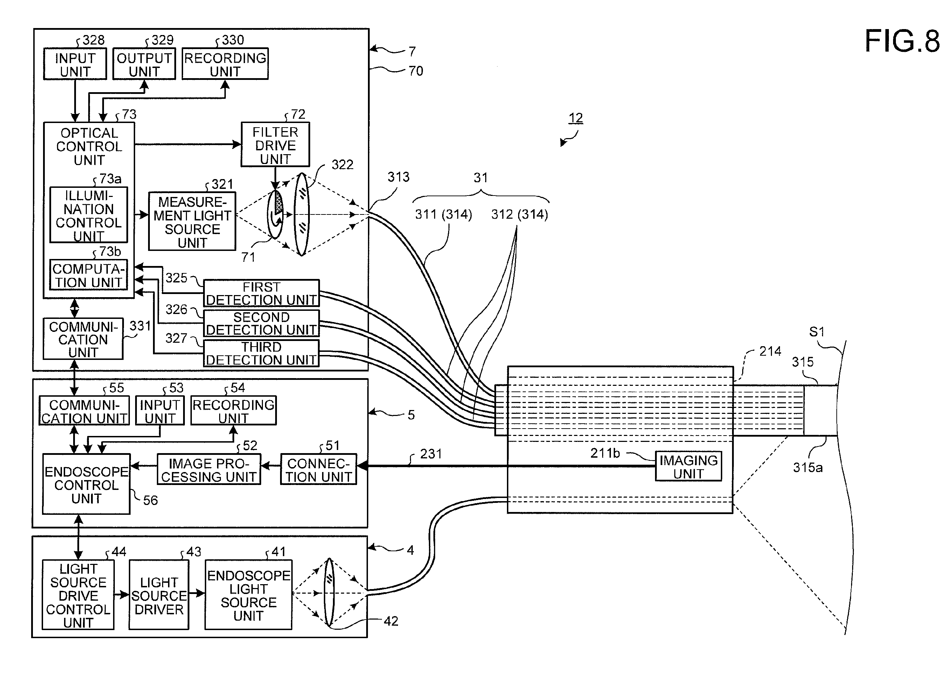

FIG. 8 is a block diagram illustrating a schematic configuration of an optical measurement device, an endoscope light source device, and a control device in an endoscope system according to a second embodiment of the present invention. An endoscope system 12 illustrated in FIG. 8 includes the endoscope light source device 4, the control device 5, and an optical measurement device 7.

The optical measurement device 7 includes the measurement probe 31 and a main body unit 70. The main body unit 70 includes a rotary filter 71, a filter drive unit 72, an optical control unit 73, the measurement light source unit 321, the condenser lens 322, the first detection unit 325, the second detection unit 326, the third detection unit 327, the input unit 328, the output unit 329, the recording unit 330, and the communication unit 331.

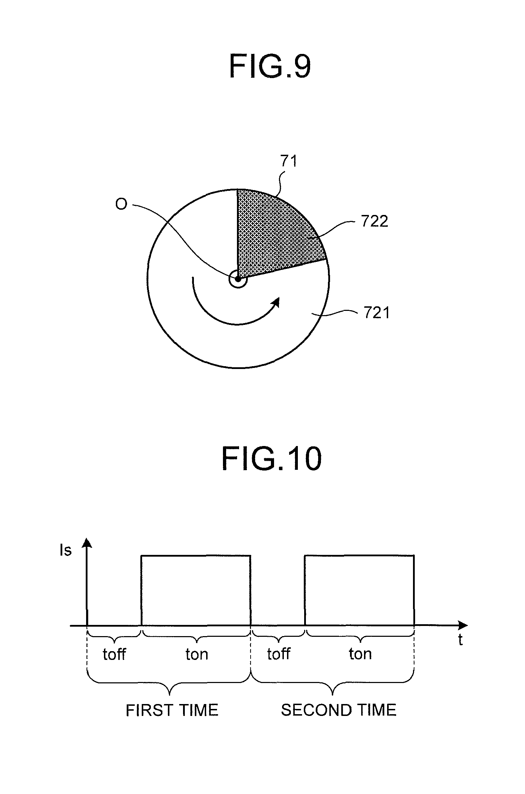

The rotary filter 71 is rotatably arranged on a light path of the measurement light emitted from the measurement light source unit 321 to periodically block out the measurement light radiated from the measurement light source unit 321. FIG. 9 is a diagram illustrating a schematic configuration of the rotary filter 71. As illustrated in FIG. 9, the rotary filter 71 has a disc shape. The rotary filter 71 has a transmission unit 721 through which the measurement light passes and a shield unit 722 that blocks out the measurement light. The transmission unit 721 is formed from a transparent member. The shield unit 722 is formed from a light-shielding member.

The filter drive unit 72 rotates the rotary filter 71 around a predetermined axis O under control of the optical control unit 73. The filter drive unit 72 is composed of a stepping motor or the like.

The optical control unit 73 is composed of a CPU or the like, and controls processing operations by the components of the optical measurement device 7. The optical control unit 73 performs centralized control of operations of the optical measurement device 7 by transferring instructive information and data to the components of the optical measurement device 7. The optical control unit 73 has an illumination control unit 73a and a computation unit 73b.

The illumination control unit 73a drives the filter drive unit 72 to rotate the rotary filter 71 with predetermined periodicity, thereby to control the measurement light source unit 321 such that the emission time during which the measurement light source unit 321 emits the measurement light is longer than the stoppage time during which the measurement light source unit 321 stops the measurement light, and causes the measurement light source unit 321 to execute alternately emission and stoppage of the measurement light plural times in a consecutive manner.

The computation unit 73b uses the foregoing equations (1) and (2) to compute the average of a plurality of intensities repeatedly detected by the first detection unit 325. The computation unit 73b also uses the foregoing equations (1) and (2) to compute the average of a plurality of intensities repeatedly detected by the second detection unit 326 and the third detection unit 327, as the first detection unit 325.

In the thus configured endoscope system 1, the radiation timing for the optical measurement device 7 to radiate the measurement light during observation of the subject will be described. FIG. 10 is a diagram illustrating radiation timings for the optical measurement device 7 to radiate the measurement light during observation of the subject. Referring to FIG. 10, the horizontal axis indicates time (t), and the vertical axis indicates intensity $I_s$ of measurement light detected by the first detection unit 325.

As illustrated in FIG. 10, the illumination control unit 73a drives the filter drive unit 72 within a period of time for measurement of the living tissue S1, thereby to control the rotation timing of the rotary filter 71 such that the stoppage time $t_{off}$ during which the living tissue S1 does not radiate the measurement light is longer than the emission time $t_{on}$ during which the measurement light source unit 321 emits the measurement light. Further, the illumination control unit 73a controls the measurement light source unit 321 and the filter drive unit 72 to alternate between the stoppage time $t_{off}$ during which the measurement light source unit 321 stops the measurement light and the emission time $t_{on}$ during which the measurement light source unit 321 emits the measurement light plural times in a consecutive manner.

According to the second embodiment of the present invention described above, the illumination control unit 73a causes the measurement light source unit 321 to repeatedly execute radiation of the measurement light with a set of the stoppage time $t_{off}$ and the emission time $t_{on}$, which makes it possible to perform more precise measurement, following variations in the living tissue S1.

In addition, according to the second embodiment of the present invention, when the measurement light source unit 321 executes repeatedly plural times radiation of the measurement light with a set of the stoppage time $t_{off}$ and the emission time $t_{on}$, the computation unit 73b computes the average of a plurality of intensities detected by the first detection unit 325, thereby obtaining results with improvement of the S/N ratio.

(Third embodiment)

Next, a third embodiment of the present invention will be described. The third embodiment is different from the first embodiment described above in configuration and computation method of the optical measurement device. Thus, hereinafter, a configuration of the optical measurement device according to the third embodiment will be described, and then a method for computation performed by the optical measurement device according to the third embodiment will be described. The same components as those in the first embodiment described above will be given the same reference numerals as those in the first embodiment, and descriptions thereof will be omitted.

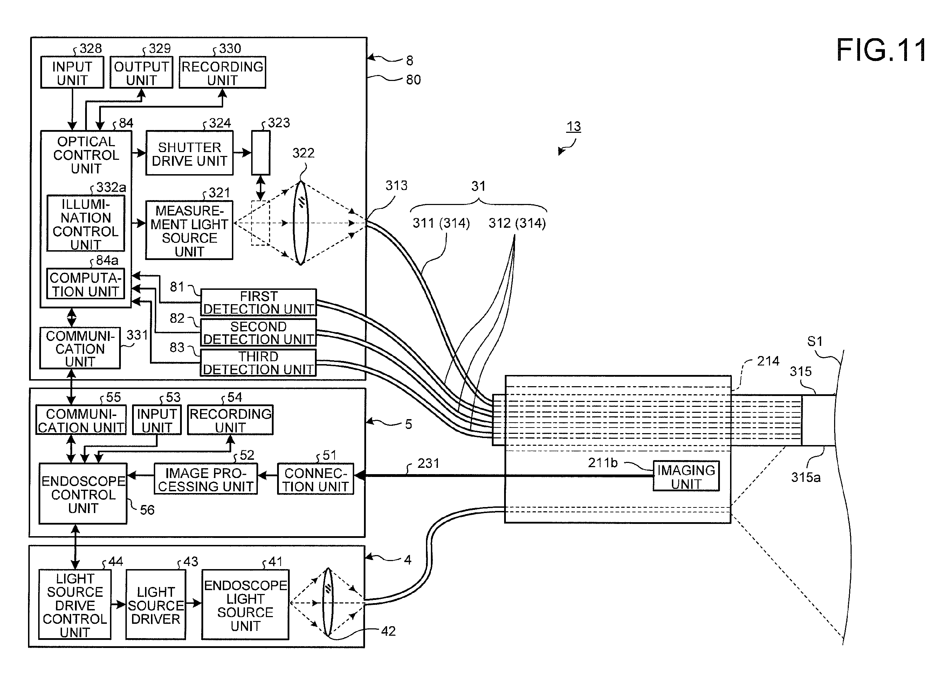

FIG. 11 is a block diagram illustrating a schematic configuration of an optical measurement device, an endoscope light source device, and a control device in an endoscope system according to the third embodiment of the present invention. An endoscope system 13 illustrated in FIG. 11 includes the endoscope light source device 4, the control device 5, and an optical measurement device 8.

The optical measurement device 8 includes the measurement probe 31 and a main body unit 80. The main body unit 80 includes the measurement light source unit 321, the condenser lens 322, the input unit 328, the output unit 329, the recording unit 330, the communication unit 331, a first spectroscope 81, a second spectroscope 82, a third spectroscope 83, and an optical control unit 84.

The first spectroscope 81 detects a spectrum of return light of the measurement light radiated from the light-receiving fibers 312 of the measurement probe 31, entered to the living tissue S1 via the measurement probe 31, and reflected and/or scattered from the living tissue S1, and then outputs the results of detection to the optical control unit 84. The first spectroscope 81 is composed of a spectroscope.

The second spectroscope 82 and the third spectroscope 83 are configured in the same manner as the first spectroscope 81, and detects a spectrum of return light of the measurement light entered via the measurement probe 31 and reflected and/or scattered from the living tissue S1, and then outputs the results of detection to the optical control unit 84.

The optical control unit 84 is composed of a CPU or the like, and controls processing operations by respective components of the optical measurement device 8. The optical control unit 84 performs centralized control of operations of the optical measurement device 8 by transferring instructive information and data to the components of the optical measurement device 8. The optical control unit 84 has the illumination control unit 332a and a computation unit 84a.

The computation unit 84a computes spectroscopic characteristics as optical characteristics of the living tissue S1, based on the spectrum of return light of the measurement light input from the first spectroscope 81, the second spectroscope 82, and the third spectroscope 83.

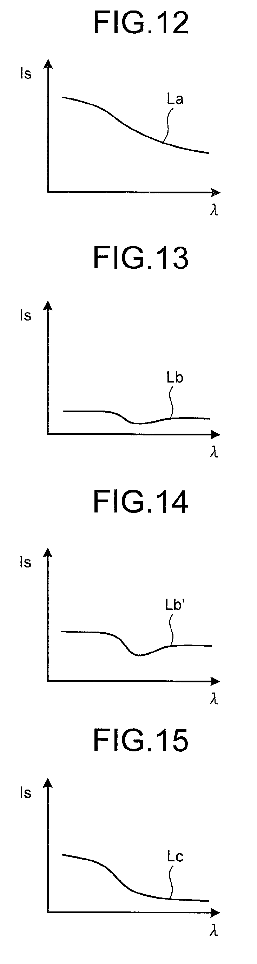

In the thus configured endoscope system 13, a method for computing by the optical measurement device 8 spectroscopic characteristics as optical characteristics of the subject will be described. FIG. 12 is a diagram illustrating a schematic spectrum $L_a$ detected during the emission time $t_{on}$ of the measurement light source unit 321 by the first spectroscope 81. FIG. 13 is a diagram illustrating a schematic spectrum $L_b$ detected during the stoppage time $t_{off}$ of the measurement light source unit 321 by the first spectroscope 82. FIG. 14 is a diagram illustrating a schematic spectrum $L_b$, of return light of illumination light radiated from the endoscope light source device 4 computed by the computation unit 84a. FIG. 15 is a diagram illustrating a schematic spectrum $L_c$ of only measurement light computed by the computation unit 84a. In FIGS. 12 to 15, the horizontal axis indicates wavelength ($\lambda$), and the vertical axis indicates detected intensity ($I_S$).

As illustrated in FIGS. 12 to 14, the computation unit 84a computes a return light spectrum $I\_B(\lambda)$ of the illumination light radiated from the endoscope light source device 4, based on a spectrum $I\_t_{on}(\lambda)$ and a spectrum $I\_t_{off}(\lambda)$ detected by the first spectroscope 81 during the emission time $t_{on}$ and the stoppage time $t_{off}$ of the measurement light source unit 321, the emission time $t_{on}$, and the stoppage time $t_{off}$, by the following equation (3):

$$I\_B(\lambda) = I\_t_{off}(\lambda) \times (t_{on}/t_{off}) \qquad (3)$$

Subsequently, as illustrated in FIG. 15, the computation unit 84a computes a spectrum $I\_C(\lambda)$ of return light of only the measurement light from the measurement light source unit 321, by the following equation (4):

$$I\_C(\lambda) = I\_t_{on}(\lambda) - I\_t_{off}(\lambda) \times (t_{on}/t_{off}) \qquad (4)$$
$$= I\_t_{on}(\lambda) - I\_B(\lambda)$$

As in the foregoing, the computation unit 84a uses the foregoing equations (3) and (4) to compute spectroscopic characteristics as optical characteristics of the living tissue S1. The computation unit 84a performs the same computation on the results of detection input from the second spectroscope 82 and the third spectroscope 83.

According to the third embodiment of the present invention described above, it is possible to measure optical characteristics of the living tissue S1 by spectroscopic characteristics.

(Modification example 1 of the third embodiment)

In the third embodiment, the illumination control unit 332a may set the emission time $t_{on}$ during which the measurement light source unit 321 emits the measurement light and the stoppage time $t_{off}$ during which the measurement light source unit 321 stops the measurement light, on the basis of the S/N ratio. For example, the illumination control unit 332a sets the emission time $t_{on}$ during which the measurement light source unit 321 emits the measurement light and the stoppage time $t_{off}$ during which the measurement light source unit 321 stops the measurement light such that the S/N ratios of the detected spectrum $I\_t_{on}(\lambda)$ and the return light spectrum $I\_B(\lambda)$ are almost equal to each other (for the details, refer to Japanese Patent Application Laid-open No. 2000-14629, equation (1), for example).

According to a modification example 1 of the third embodiment of the present invention described above, in the case of shortening the stoppage time $t_{off}$ during which the measurement light source unit 321 stops the measurement light, the optimum stoppage time $t_{off}$ can be set to prevent that a noise content is included in the detected spectrum $I\_t_{on}(\lambda)$. As a result, it is possible to obtain the results of measurement of the living tissue S1 at the optimum S/N ratio.

In the modification example 1 of the third embodiment of the present invention, spectroscopic spectrum measurement is performed as an example. The same operations can be also set in the foregoing embodiments 1 and 2.

(Fourth embodiment)

Next, a fourth embodiment of the present invention will be described. The fourth embodiment is different from the first embodiment described above in configuration and illumination method of the optical measurement device. Thus, hereinafter, a configuration of the optical measurement device according to the fourth embodiment will be described, and then a method for computation performed by the optical measurement device according to the fourth embodiment will be described. The same components as those in the first embodiment described above will be given the same reference numerals as those in the first embodiment, and descriptions thereof will be omitted.

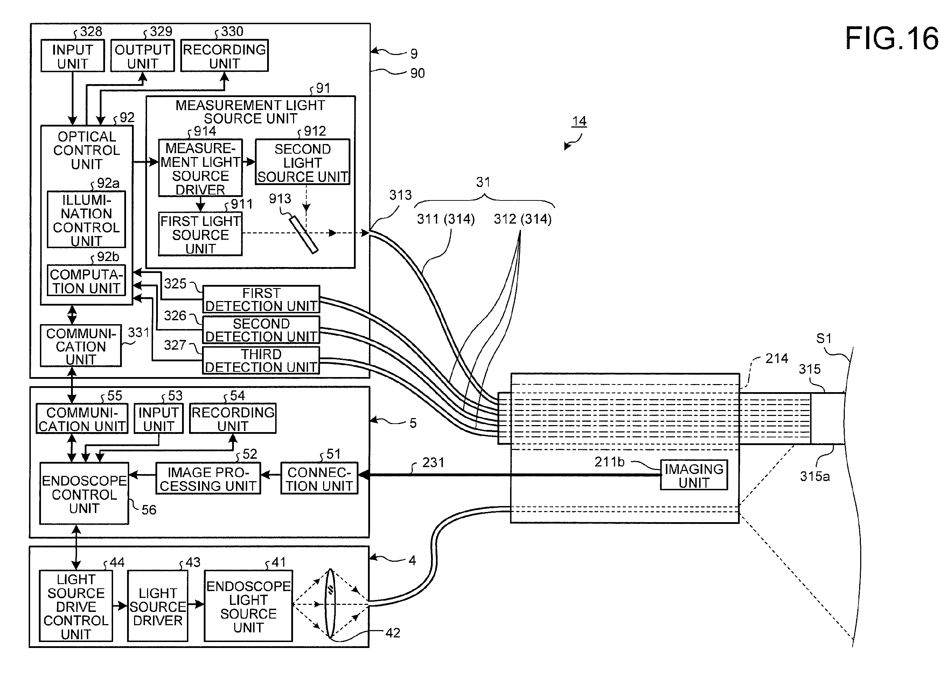

FIG. 16 is a block diagram illustrating a schematic configuration of an optical measurement device, an endoscope light source device, and a control device in an endoscope system according to the fourth embodiment of the present invention. An endoscope system 14 illustrated in FIG. 16 includes the endoscope light source device 4, the control device 5, and an optical measurement device 9.

The optical measurement device 9 includes the measurement probe 31 and a main body unit 90. The main body unit 90 includes a measurement light source unit 91, an optical control unit 92, the first detection unit 325, the second detection unit 326, the third detection unit 327, the input unit 328, the output unit 329, the recording unit 330, and the communication unit 331.

The measurement light source unit 91 has a first light source unit 911, a second light source unit 912, a mirror 913, and a measurement light source driver 914.

The first light source unit 911 emits measurement light toward the measurement probe 31. The first light source unit 911 is composed of a white LED and a condenser lens or the like.

The second light source unit 912 emits measurement light toward the measurement probe 31. The second light source unit 912 is composed of a white LED and a condenser lens or the like.

The mirror 913 transmits the measurement light radiated from the first light source unit 911 and reflects the measurement light radiated from the second light source unit 912 toward the measurement probe 31. The mirror 913 is composed of a half mirror or the like.

The measurement light source driver 914 supplies predetermined electric power to the first light source unit 911 or the second light source unit 912 under control of the optical control unit 92, thereby causing the first light source unit 911 or the second light source unit 912 to emit the measurement light toward the measurement probe 31.

The optical control unit 92 is composed of a CPU or the like, and controls processing operations by respective components of the optical measurement device 9. The optical control unit 92 performs centralized control of operations of the optical measurement device 9 by transferring instructive information and data to the components of the optical measurement device 9. The optical control unit 92 has an illumination control unit 92a and a computation unit 92b.

The illumination control unit 92a controls the measurement light source driver 914 in a period of time for measurement of the living tissue S1, such that the stoppage time during which the measurement light source unit 91 does not radiate the measurement light is the longest, followed by the emission time during which the first light source unit 911 radiates the measurement light, and the emission time during which the second light source unit 912 emits the measurement light.

The computation unit 92b computes optical characteristics of the living tissue S1 based on the results of detection input from the first detection unit 325, the second detection unit 326, and the third detection unit 327.

In the thus configured endoscope system 14, emission timings for the optical measurement device 9 to radiate the measurement light during observation of the subject and a method for computation by the computation unit 92b will be described.

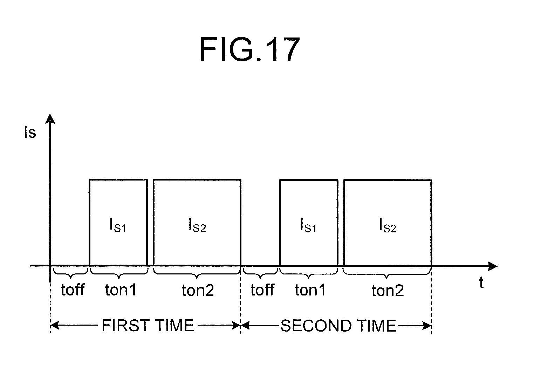

FIG. 17 is a diagram illustrating emission timings for the optical measurement device 9 to radiate measurement light. In FIG. 17, the horizontal axis indicates time (t), and the vertical axis indicates detected intensity $I_s$ of the measurement light.

As illustrated in FIG. 17, the illumination control unit 92a controls the measurement light source driver 914 in a period of time for measurement of the living tissue S1, such that the stoppage time $t_{off}$ during which no measurement light is emitted is the longest, followed by emission time $t_{on1}$ during which the first light source unit 911 emits the measurement light and emission time $t_{on2}$ during which the second light source unit 912 emits the measurement light. The illumination control unit 92a further causes the measurement light source driver 914 to execute repeatedly the stoppage time $t_{off}$ during which no measurement light is emitted, the emission time $t_{on1}$ during which the first light source unit 911 emits the measurement light and the emission time $t_{on2}$ during which the second light source unit 912 emits the measurement light.

Next, a method for computation by the computation unit 92b will be described. If it is assumed that the intensity detected by the first detection unit 325 during the stoppage time $t_{off}$ is designated as $I\_t_{off}$, the intensity detected by the first detection unit 325 during the emission time $t_{on1}$ is designated as $I\_t_{on1}$ , and the intensity detected by the first detection unit 325 during the emission time $t_{on2}$ is designated as $I\_t_{on2}$, detected intensities I_C1 and I_C2 of return light from the first light source unit 911 and the second light source unit 912 are computed by the following equations (5) and (6):

$$I\_C1 = I\_t_{on1} - I\_t_{off} \times (t_{on1}/t_{off}) \qquad (5)$$

$$I\_C2 = I\_t_{on2} - I\_t_{off} \times (t_{on2}/t_{off}) \qquad (6)$$

As in the foregoing, the computation unit 92b computes detected intensities of return light of only measurement light from the first light source unit 911 and the second light source unit 912, using the foregoing equation (5) or (6). The computation unit 92b performs the same computation on the results of detection by the second detection unit 326 and the third detection unit 327.

According to the fourth embodiment of the present invention described above, even if the first light source unit 911 and the second light source unit 912 separately radiate measurement light, it is possible to compute detected intensities of return light from the first light source unit 911 and the second light source unit 912.

Although the emission time $t_{on1}$ is shorter than the emission time $t_{on2}$ in the foregoing fourth embodiment, the emission time $t_{on1}$ may be longer than the emission time $t_{on2}$.

Thus, the present invention may include various embodiments not described herein, and can be subjected to various design changes and the like within the scope of technical ideas specified by the claims.

Reference Signs List

1, 10, 12, 13, 14 Endoscope system
2 Endoscope device
3, 7, 8, 9, 11 Optical measurement device
4 Endoscope light source device
5 Control device
6 Display device
21 Insertion potion
22 Operating unit
23 Universal cord
31 Measurement probe
32, 70, 80, 90, 110 Main body unit
41 Endoscope light source unit
42 Condenser lens
43 Light source driver
44 Light source drive control unit
51 Connection unit
52 Image processing unit
53, 328 Input unit
54, 330 Recording unit
55, 331 Communication unit
56 Endoscope control unit
71 Rotary filter
72 Filter drive unit
73, 84, 92, 112, 332 Optical control unit
73a, 92a, 112a, 332a Illumination control unit
73b, 84a, 92b, 332b Computation unit
81 First spectroscope
82 Second spectroscope
83 Third spectroscope
91, 111, 321 Measurement light source unit
211b Imaging unit
211 Distal end portion
212 Curve portion
222 Treatment tool insertion portion
311 Illumination fiber
312 Light-receiving fiber
313 Proximal end portion
314 Flexible portion
315 Distal end portion
315a Rod
322 Condenser lens
323 Shutter
324 Shutter drive unit
325 First detection unit
326 Second detection unit
327 Third detection unit
329 Output unit
911 First light source unit
912 Second light source unit
913 Mirror
914 Measurement light source driver

**Claims**

1. An optical measurement device configured to be inserted into an inside of a subject via an insertion portion of an endoscope radiating observation light for observing a target from a distal end of the insertion portion to be inserted into the inside of the subject to shoot the inside of the subject, and to measure optical characteristics of a living tissue in the subject, the optical measurement device comprising:

   a measurement light source unit that emits measurement light including at least part of a wavelength band of the observation light radiated by the endoscope;
   a measurement probe including
   an illumination fiber configured to radiate the measurement light emitted from the measurement light source unit to the living tissue, and
   a light-receiving fiber configured to receive return light of the measurement light reflected and/or scattered from the living tissue;
   a detection unit that detects the return light of the measurement light received by the light-receiving fiber;
   an illumination control unit that causes the measurement light source unit to execute alternately emission and stoppage of the measurement light within the time from the start to end of the measurement by the optical measurement device, and causes the measurement light source unit to emit the measurement light such that the emission time during which the measurement light source unit emits the measurement light is longer than the stoppage time during which the measurement light source unit stops the measurement light; and
   a computation unit that removes, from an intensity of return light of the measurement light detected by the detection unit, a detected intensity of return light of the observation light radiated by the endoscope device and reflected from the living tissue, wherein
   the computation unit computes a detected intensity I_C in which the detected intensity of return light of the observation light is removed from the detected intensity of return light of the measurement light by the following equation (1):

   $$I\_C = I\_t_{on} - I\_t_{off} \times (t_{on}/t_{off}) \qquad \dots (1)$$

   where $t_{on}$ denotes the emission time, $t_{off}$ denotes the stoppage time, $I\_t_{on}$ denotes the intensity detected by the detection unit during the emission time, and $I\_t_{off}$ denotes the intensity detected by the detection unit during the stoppage time.

2. The optical measurement device according to claim 1, wherein the illumination control unit performs variable control on the emission time and the stoppage time according to the type of the living tissue.

3. The optical measurement device according to claim 1 or 2, wherein
   the illumination control unit causes the measurement light source unit to execute alternately emission and stoppage of the measurement light, and
   the computation unit computes the average value of a plurality of intensities repeatedly detected by the detection unit.

4. The optical measurement device according to any of claims 1 to 3, wherein
   the optical characteristics are a spectrum of return light of the measurement light, and
   the detection unit has a spectroscope that detects the spectrum of return light of the measurement light.

5. The optical measurement device according to any of claims 1 to 4, wherein the illumination control unit causes the measurement light source unit to emit the measurement light during the emission time and the stoppage time where the intensity detected by the detection unit during the emission time and the detected intensity of return light of the observation light from the endoscope are equal in S/N ratio.

6. The optical measurement device according to any of claims 1 to 5, wherein
   the light-receiving fiber includes a plurality of light-receiving channels that receive return light of the measurement light at different angles, and
   the detection unit includes a plurality of detection units that detect the return light of the measurement light received by the plurality of light-receiving channels.

# FIG.1

FIG.2

# FIG.3

# FIG.4

Is

I_A
(I_ton)

(a)

(b)

# FIG.5

Is

(a')

I_B
(I_toff)

# FIG.6

Is

I_C

(b)

# FIG.7

# FIG.8

# FIG.9

# FIG.10

# FIG.11

EP 2 823 750 A1

## FIG.12

## FIG.13

## FIG.14

## FIG.15

FIG.16

# FIG.17

## INTERNATIONAL SEARCH REPORT

| | International application No. |
|---|---|
| | PCT/JP2013/056187 |

**A. CLASSIFICATION OF SUBJECT MATTER**
*A61B1/06*(2006.01)i

According to International Patent Classification (IPC) or to both national classification and IPC

**B. FIELDS SEARCHED**

Minimum documentation searched (classification system followed by classification symbols)
A61B1/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2000-14629 A  (Olympus Optical Co., Ltd.),<br>08 November 2000 (08.11.2000),<br>paragraphs [0148] to [0151]<br>(Family: none) | 1-4<br>6 |
| Y | JP 9-56662 A  (Hitachi, Ltd.),<br>04 March 1997 (04.03.1997),<br>paragraph [0006]; fig. 2<br>(Family: none) | 6 |
| A | JP 10-337274 A  (Olympus Optical Co., Ltd.),<br>22 December 1998 (22.12.1998),<br>paragraph [0199]<br>(Family: none) | 1-6 |

☐ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 June, 2013 (05.06.13) | 18 June, 2013 (18.06.13) |

| Name and mailing address of the ISA/<br>Japanese Patent Office | Authorized officer |
|---|---|
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9248281 A

- JP 2000014629 A